# EUROPÄISCHE PATENTSCHRIFT

(11) **EP 1 857 096 B1**
(45) Veröffentlichungstag und Bekanntmachung des Hinweises auf die Patenterteilung: **05.12.2012**
(21) Anmeldenummer: 07107663.2
(22) Anmeldetag: 08.05.2007
(51) Int. Cl.: A61K 8/29, A61K 8/49, A61K 8/81, A61Q 17/04

(54) **Sprühbares Sonnenschutzmittel**
Sprayable sun protection agent
Protection solaire pouvant être vaporisée

(30) Priorität: 15.05.2006 DE 202006007976 U
(43) Veröffentlichungstag der Anmeldung: 21.11.2007
(73) Patentinhaber: Beiersdorf AG, 20253 Hamburg (DE)
(72) Erfinder: Windisch, Björna Dr., 22607, Hamburg (DE); Weingarz, Yvonne, 22763 Hamburg (DE)

(56) Entgegenhaltungen:
- EP-A1- 1 284 129
- EP-A2- 1 077 062
- DE-A1- 10 106 133
- DE-A1- 19 938 757

## Beschreibung

Die vorliegende Erfindung betrifft eine kosmetische Zubereitung enthaltend Titandioxidpigmente, ein Verdickersystem aus polymeren Verdickungsmitteln, welche jeweils quervernetzt sind, sowie 2-Phenylbenzimidazol-5-sulfonsäure und/oder deren Natrium-, Kalium oder Triethanolaminsalze, welches dadurch gekennzeichnet ist, dass das Verhältnis von Titandioxidpigmenten zu 2-Phenylbenzimidazol-5-sulfonsäure und/oder deren Natrium-, Kalium oder Triethanolaminsalze (Gesamtmenge) 1:1,2 bis 1,2:1 beträgt.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

So gibt es beispielsweise bis heute keine dünnflüssig sprühbaren Sonnenschutzmittel, die einen Lichtschutzfaktor (SPF) von mindestens 50 aufweisen. Zubereitungen mit hohem Lichtschutzfaktor zeigen in der Regel eine zu hohe Viskosität, als dass sie mit einem in der Kosmetik üblichen Sprühapplikator versprüht werden können. Dies liegt nicht zuletzt an den hohen Einsatzkonzentrationen von Titandioxid, welches gewöhnlich benötigt wird, um hohe Lichtschutzfaktoren zu erhalten. Reduziert man die Viskosität dieser Zubereitungen, so sind die Formulierungen nicht mehr temperatur- und lagerstabil und neigen zur Phasentrennung. Darüber hinaus wird der Lichtschutzfaktor drastisch reduziert.

Eine der am weitesten verbreiteten Grundlagen für kosmetische Zubereitungen stellen die O/W-Emulsionen dar. Dies liegt nicht zuletzt an den angenehm leichten, fettarmen Sensorik dieser Formulierungen, welche schnell und nahezu vollständig in die Haut einziehen. Emulsionen sind jedoch bekanntermaßen äußerst elektrolytempfindlich und neigen bei erhöhtem Elektrolytgehalt zur Phasentrennung. Aus diesem Grunde ist es nach dem Stande der Technik bisher nicht möglich gewesen, den in Elektrolytform vorliegenden UV-Lichtschutzfilter Phenylbenimidazol-5-sulfonsäure bzw. dessen Natrium-, Kalium oder Triethanolamoniumsalze in größeren Konzentrationen in emulsionsförmige Zubereitungen einzuarbeiten.

Bei kosmetischen Zubereitungen in Sprayform kommt es nicht allein darauf an, dass die Zubereitung durch den Sprühapplikator auf die Haut aufgetragen wird. Wichtig ist darüber hinaus die Art der Verteilung der Zubereitung auf der Haut; das so genannte "Sprühbild". Denn naturgemäß erwartet der Anwender solcher Zubereitungen von dem Produkt, dass er es nach dem Aufsprühen auf die Haut nicht mehr zusätzlich auf derselben durch Verreiben verteilen muss. Das Sprühbild kosmetischer Zubereitungen hängt aber nicht allein von der Ausgestaltung des Sprühapplikators ab. Einen zusätzlichen Einfluss auf das Sprühbild hat auch die Zusammensetzung der Zubereitung selbst. Hier kommt es insbesondere darauf an, ob sich die Spraytröpfchen während und nach dem Aufsprühen schnell wieder zu größeren Tropfen vereinigen oder nicht. Die meisten Zubereitungen des Standes der Technik (insbesondere wenn es sich um Zubereitungen mit relativ hohem Lichtschutzfaktor handelt) zeigen ein ungleichmäßiges Sprühbild, bei welchem die Spraytröpfchen schnell agglomerieren.

Eine weitere Anforderung, welche an die sprühbare Zubereitung gestellt wird, besteht darin, dass die Zubereitung nach dem Auftragen "ortsfest" auf der Haut verbleibt und nicht etwa durch zerfließen sich ungleichmäßig auf der Haut verteilt und unter Umständen Körperpartien und Kleidungsstücke kontaminiert, die mit der Zubereitung nicht in Kontakt kommen sollten. Aus diesem Grunde muss die Zubereitung ein thixotropes Verhalten aufweisen und einerseits unter Scherung (beim Sprühvorgang) hinreichdend dünnflüssig sein um überhaupt versprüht werden zu können, andererseits nach dem Auftragen auf die Haut so zähflüssig sein, dass sie nicht selbsttätig zerfließt. Dieses Kriterium ist jedoch insbesondere bei Zubereitungen mit hohem Lichtschutzfaktor bisher nur unvollständig erfüllt.

Desweiteren weisen nur Produkte mit einer relativ hohen Viskosität im Ruhezustand eine sehr gute physikalische Stabilität auf, die bei sehr dünnflüssigen Zubereitungen nicht ausreichend gegeben ist. Die hochviskosen Zubereitungen müssen dann ein entsprechend starkes thixotropes Verhalten aufweisen um versprüht werden zu können. Die Zubereitungen des Standes der Technik weisen jedoch meist ein nur unzureichendes thixotropes Verhalten auf und lassen sich bei hoher Viskosität nicht mehr richtig versprühen.

Es war daher die Aufgabe der vorliegenden Erfindung die Mängel des Standes der Technik zu beseitigen.

Insbesondere war es die Aufgabe der vorliegenden Erfindung, eine temperatur- und lagerstabile dünnflüssig-sprühbare, kosmetische Sonnenschutzzubereitung zu entwickeln, die einen hohen SPF (von mindestens 50) aufweist. Darüber hinaus sollte die Zubereitung eine hohe UV-A-Balance (von mindestens 20) aufweisen.

Die Zubereitungen sollten sich leicht versprühen lassen, ein gleichmäßiges Sprühbild auf der Haut hinterlassen, ortsfest auf der Haut verbleiben und schnell in die Haut einziehen.

Überraschend gelöst werden die Aufgaben durch eine kosmetische Zubereitung enthaltend Titandioxidpigmente,
ein Verdickersystem aus polymeren Verdickungsmitteln, welche jeweils quervernetzt sind, 2-Phenylbenzimidazol-5-sulfonsäure und/oder deren Natrium-, Kalium oder Triethanolaminsalze, wobei
das Verhältnis von Titandioxidpigmenten zu 2-Phenylbenzimidazol-5-sulfonsäure und/oder deren Natrium-, Kalium oder Triethanolaminsalze (Gesamtmenge) 1:1,2 bis 1,2:1 beträgt,
die Gesamtmenge an Titandioxidpigmenten und 2-Phenylbenzimidazol-5-sulfonsäure und/oder deren Natrium-, Kalium oder Triethanolaminsalzen 2,5 bis 3,5 Gewichts-%,
bezogen auf das Gesamtgewicht der Zubereitung beträgt und die Zubereitung das Verdickersystem in einer Konzentration von 0,35 bis 0,45 Gewichts-%, bezogen auf das Gesamtgewicht der Zubereitung, enthält, dadurch gekennzeichnet, dass die Titandioxidpigmente mit Trimethoxycaprylylsifan beschichtet sind.

Zwar kennt der Stand der Technik eine Reihe von Veröffentlichungen zu Titandioxid, doch konnten diese Schriften nicht den Weg zur vorliegenden Erfindung weisen:

So offenbart die DE 4303983 weder die Kombination aus Titandioxidpigmenten 2-Phenylbenimidazol-5-sulfonsäure noch das erfindungsgemäße Verdickersystem.

Die EP 0683662 offenbart weder das erfindungsgemäße Verdickersystem noch die Kombination aus Titandioxidpigmenten und 2- Phenylbenimidazol-5-sulfonsäure im Verhältnis 1:1,2-1,2:1.

Die EP 0610926 offenbart weder die Kombination aus Titandioxidpigmenten 2-Phenylbenimidazol-5-sulfonsäure noch das erfindungsgemäße Verdickersystem.

Die DE 19725087 offenbart weder die Kombination aus Titandioxidpigmenten und 2-Phenylbenimidazol-5-sulfonsäure noch das erfindungsgemäße Verdickersystem.

Die DE 19817296 offenbart weder die Kombination aus Titandioxidpigmenten und 2-Phenylbenimidazol-5-sulfonsäure noch das erfindungsgemäße Verdickersystem.

Die DE 19846771 offenbart weder die Kombination aus Titandioxidpigmenten und 2-Phenylbenimidazol-5-sulfonsäure noch das erfindungsgemäße Verdickersystem.

Die DE 19938756 offenbart nicht das erfindungsgemäße Verdickersystem.

Die DE 19938757 offenbart nicht das erfindungsgemäße Verdickersystem. Die Titandioxidpigmente sind mit Simethicon beschichtet.

Die DE 10106133 offenbart nicht das erfindungsgemäße Verdickersystem.

Die EP 1077062 offenbart eine dünnflüssige, sprühbare kosmetische Zubereitungmit einem vernetzten Verdicker (Pemulen TR1 und TR2) sowie Phenylbenzimidazolsulfonsäure. Diese Schrift offenbart jedoch kein Titandioxid, das mit Trimethoxycaprylysilan beschichtet ist.

Es ist erfindungsgemäß bevorzugt, wenn die Gesamtmenge an Titandioxidpigmenten und 2.

Phenylbenzimidazol-5-sulfonsäure und/oder deren Natrium-, Kalium oder Triethanolaminsalzen von 2,8 bis 3,2 Gewichts-%, bezogen auf das Gesamtgewicht der Zubereitung beträgt.

Erfindungsgemäß vorteilhaft ist es, wenn die erfindungsgemäße Zubereitung einen Lichtschutzfaktor (SPF) von größer oder gleich 50 aufweist, wobei ein Lichtschutzfaktor von größer oder gleich 60 erfindungsgemäß bevorzugt ist.

Der SPF wird dabei erfindungsgemäß mit der in-vivo-Methode gemessen, die durch die "International Sun Protection Factor (SPF) Test Method. CTFA South Africa, COLIPA and JCIA, February 2003" festgelegt ist.

Erfindungsgemäß vorteilhafte Ausführungsformen der vorliegenden Erfindung sind dadurch gekennzeichnet, dass die Zubereitung eine Viskosität von 300 bis 2000 mPaS aufweist. Dabei ist eine Viskosität von 500 bis 1500 mPas erfindungsgemäß bevorzugt.

Die erfindungsgemäße Viskosität wurde mit einem Haake ViscoTester 02 Viskosimeter gemessen. (Dieser Viskosimetertyp besteht aus einem rotierenden Zylinder und einem ruhenden Messbecher. Über die Rotationswiderstandskraft der Formulierung ermittelt das Gerät die Viskosität der Probe. Drehzahl (rpm)= 62,5. Die Viskosität wird nach 30 s. ermittelt. Messtemperatur =25°C).

Die erfindungsgemäβen zubereitungen sind dadurch gekennezeichnet, class die Titandioxid-pigmente hydrophob mit Trimethoxycaprylsilan deschichtet sind.

Es ist erfindungsgemäß vorteilhaft, wenn die erfindungsgemäßen Titandioxidpigmente eine durchschnittliche Teilchengröße von 35 nm x 29 nm aufweisen.

Erfindungsgemäß besonders bevorzugt werden als Polymere im Verdickersystem quervernetzte Acrylat/C10-30 Alkylacrylate (INCI: Acrylates/C10-30 Alkyl Acrylate Crosspolymer) eingesetzt. Dabei ist es insbesondere bevorzugt, Carbopol 1382 (Goodrich) und Pemulen TR-1 (Goodrich) als quervernetzen Polymere einzusetzen.

Die erfindungsgemäße Zubereitung kann neben den erfindungsgemäßen UV-Lichtschutzfiltern weitere UV-Lichtschutzfilter enthalten. Dabei können ein oder mehrere weitere UV-Filter gewählt werden aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bomylidenmothyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher, 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazane); 2,4,6-Tribiphenyl-4-yl-1,3,5-triazin; Merocyanine; Zinkoxid. Diese zusätzlichen Filter können in einer Konzentration von 0,01 bis 40 Gewichts-% und bevorzugt in einer Konzentration von 1 bis 20 Gewichts-%, bezogen auf das Gesamtgewicht der Formulierung in der Zubereitung enthalten sein.

Allerdings ist es erfindungsgemäß bevorzugt, wenn die erfindungsgemäße Zubereitung frei ist von 3-(4'-Methyl)benzylidenboran-2-on (INCI: 4-Methylbenzylidene Camphor).

Es ist erfindungsgemäß vorteilhaft, wenn die erfindungsgemäße Zubereitung eine UVA-Balance von größer oder gleich 20 aufweist. Eine UV-A-Balance von größer oder gleich 30 ist erfindungsgemäß bevorzugt. Besonders bevorzugt weist die erfindungsgemäße Zubereitung eine UV-A-Balance von größer oder gleich 40 auf. Die erfindungsgemäße UV-A-Balance wird dabei erfindungsgemäß nach DIN 67502 bestimmt.

Es ist erfindungsgemäß besonders bevorzugt, wenn die erfindungsgemäße Zubereitung in Form einer Öl-in-Wasser-Emulsion vorliegt.

Erfindungsgemäß vorteilhaft enthält die erfindungsgemäße Zubereitung dabei keine Stearinsäure, Isostearinsäure, kein Trilaureth-4 Phosphat und kein Cetylphosphat.

Die erfindungsgemäße Zubereitung kann erfindungsgemäß vorteilhaft Feuchthaltemittel enthalten. Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Schaumstabilisatoren, Elektrolyte, Selbstbräuner.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP) 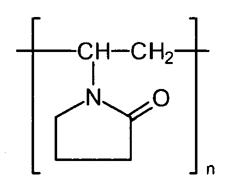 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole. Ebenfall vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase Verbindungsgen aus der Gruppe der Dialkylether und Dialkylcarbonate, wie z. B. Dicaprylylether und/oder Dicaprylylcarbonat enthalten.

Es ist jedoch erfindungsgemäß bevorzugt, wenn die erfindungsgemäße Zubereitung frei ist von Caprylylglycol.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*HallstarAB*) und/oder Diethylhexylnaphthalat (*Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Ein Gehalt an Silikonölen (z.B. Dimethicon, Cyclomethicon) in der Zubereitung ist erfindungsgemäß vorteilhaft.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, dass die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate (z.B. Tocopherylacetat) sowie Vitamin A und dessen Derivate (z.B. Retinylpalmitat).

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure und/oder Licochalcon A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen) und ermüdete Haut. Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Erfindungsgemäß vorteilhaft weist die erfindungsgemäße Zubereitung einen pH-Wert von 6 bis 8,5 auf. Dieser kann durch die herkömmlichen Säuren, Basen und Puffersysteme eingestellt werden.

Erfindungsgemäß ist auch ein Sprühapplikator enthaltend die erfindungsgemäße Zubereitung. Als Sprühapplikator kann dabei sowohl eine Aerosoldose, ein "bag-in-can"-System, bei welchem die Zubereitung in einem Beutel in einer Dose mit Überdruck aufbewahrt wird, oder ein Pumpspendersystem verwendet werden.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Zur Anwendung werden die erfindungsgemäßen kosmetischen Zubereitungen in der für Kosmetika üblichen Weise auf die Haut und/oder die Haare in ausreichender Menge aufgebracht.

Erfindungsgemäß ist auch das Verfahren zur Herstellung der erfindungsgemäßen kosmetischen Zubereitung welches dadurch gekennzeichnet ist, dass zunächst die Ölphase und die Wasserphase getrennt hergestellt und anschließend vereinigt werden und wobei das erfindungsgemäße Verdickersystem vor der Vereinigung der beiden Phasen der Wasserphase zugesetzt wird.

Erfindungsgemäß sind auch die Zubereitungen, die nach dem erfindungsgemäßen Verfahren hergestellt sind.

### Vergleichsversuche

Die erfindungsgemäßen Zubereitungen zeichnen sich insbesondere durch eine hohe Stabilität bei gleichzeitig relativ geringer Viskosität aus, bei welcher die Zubereitungen ein thixotropes Fließverhalten aufweisen, wodurch die Zubereitungen sprühbar werden.

Die folgenden Tabellen zeigen die Abhängigkeit der Sprühbarkeit und Stabilität der Zubereitungen von der Einsatzkonzentration der UV-Filter Titandioxidpigmente, 2-Phenylbenzimidazol-5-sulfonsäure und vom Gehalt an Verdickersystem. Es wurden in eine erfindungsgemäße Basisformulierung unterschiedliche Mengen der Filter eingearbeitet:

| | Versuch | Versuch | Versuch | Versuch | Versuch | Versuch |
|---|---|---|---|---|---|---|
| | 1 | 2 | 3 | 4 | 5 | 6 |
| 2-Phenylbenz-imidazol-5-sulfonsäure-gehalt | 0,5 | 1,0 | 1,5 | 2 | 2 | 2 |
| Titandioxidgehalt | 1,5 | 1,5 | 1,5 | 2 | 2,5 | 1,5 |
| Sprühbarkeit | - | o | + | - | - | + |
| Stabilität | + | + | + | o | + | - |
| SPF >50 | - | - | + | + | + | + |

| | | | | | | |
|---|---|---|---|---|---|---|
| Bewertung: + =sprühbar, stabil - = nicht sprühbar, nicht stabil o = begrenzt sprühbar, begrenzt stabil Konzentrationsangaben in Gewichts-% der Zubereitung | | | | | | |

| | Versuch 7 | Versuch 8 | Versuch 9 |
|---|---|---|---|
| Konzentration Verdickersystem | 0,25 | 0,40 | 0,55 |
| Sprühbarkeit | + | + | - |
| Stabilität | - | + | + |

| | | | |
|---|---|---|---|
| Bewertung: + =sprühbar, stabil - = nicht sprühbar, nicht stabil o = begrenzt sprühbar, begrenzt stabil Konzentrationsangaben in Gewichts-% der Zubereitung | | | |

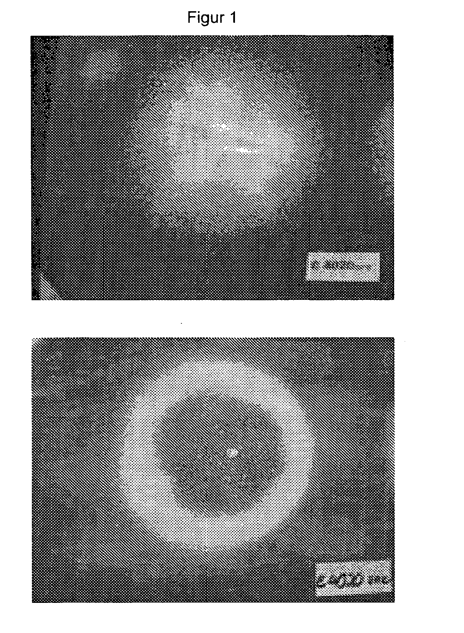

Abhängigkeit des Sprühbildes von der Einsatzkonzentration vom Gehalt an 2-Phenylbenzimidazol-5-sulfonsäure (Figur 1):
Bild oben: Erfindungsgemäße Zubereitung mit 2-Phenylbenz-imidazol-5-sulfonsäure
Bild unten: Erfindungsgemäße Zubereitung ohne 2-Phenylbenz-imidazol-5-sulfonsäure

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI-Name(n)** | **m[%]** | **m[%]** | **m[%]** | **m[%]** | **m[%]** |
|---|---|---|---|---|---|
| Acrylates/C10-30 Alkyl Acrylate Crosspolymer | 0,35 | 0,35 | 0,40 | 0,40 | 0,40 |
| Alcohol Denat. | 2,00 | 3,00 | 4,00 | 4,00 | 4,00 |
| Bis-Ethyl hexyloxyphenol Methoxyphenyl Triazine | 3,50 | 3,50 | 3,50 | 3,50 | 3,50 |
| Butyl Methoxydibenzoylmethane | 4,00 | 4,50 | 4,50 | 4,50 | 4,50 |
| Butylene Glycol Dicaprylate/Dicaprate | 3,00 | 2,00 | 7,00 | 5,00 | 6,00 |
| C18-36 Acid Triglyceride | 0,50 | 0,70 | 1,00 | 1,00 | 1,50 |
| Ceteareth-20 | 0,60 | 1,00 | 1,50 | 1,00 | 2,00 |
| Diethylhexyl Butamido Triazone | 2,00 | 3,00 | 4,00 | 2,50 | 2,00 |
| Ethylhexyl Methoxycinnamate + BHT | 2,00 | 0,00 | 0,50 | 0,50 | 1,00 |
| Ethylhexyl Salicylate | 3,00 | 4,00 | 5,00 | 3,00 | 1,00 |
| Fragrance | 0,30 | 0,20 | 0,40 | 0,30 | 0,20 |
| Glycerin | 4,00 | 6,00 | 7,00 | 5,00 | 6,00 |
| Homosalate | 6,00 | 5,00 | 7,00 | 7,00 | 8,00 |
| Methylparaben | 0,30 | 0,20 | 0,30 | 0,30 | 0,10 |
| Octocrylene | 5,00 | 3,00 | 3,50 | 4,50 | 2,50 |
| Phenoxyethanol | 0,60 | 0,40 | 0,60 | 0,60 | 0,50 |
| Phenylbenzimidazole Sulfonic Acid | 1,50 | 1,50 | 1,50 | 1,50 | 1,50 |
| Propylparaben | 0,20 | 0,10 | 0,10 | 0,10 | 0,20 |
| Titanium Dioxide + Trimethoxycaprylylsilane | 1,50 | 1,50 | 1,50 | 1,50 | 1,50 |
| Tocopheryl Acetate | 0,30 | 0,50 | 1,00 | 0,50 | 1,00 |
| Trisodium EDTA | 0,50 | 0,70 | 1,00 | 1,00 | 0,60 |
| VP/Hexadecene Copolymer | 0,00 | 0,30 | 0,50 | 0,50 | 0,60 |
| Water + Sodium Hydroxide | 0,65 | 0,76 | 0,70 | 0,76 | 0,73 |
| Water | 61,0 | 57,79 | 43,50 | 52,04 | 50,67 |
| | 100,00 | 100,00 | 100,00 | 100,00 | 100,00 |

## Patentansprüche

1. Kosmetische Zubereitung enthaltend
Titandioxidpigmente,
ein Verdickersystem aus polymeren Verdickungsmitteln, welche jeweils quervernetzt sind, 2-Phenylbenzimidazol-5-sulfonsaure und/oder deren Natrium-, Kalium oder Triethanolaminsalze, wobei
das Verhältnis von Titandioxidpigmenten zu 2-Phenylbenzimidazol-5-sulfonsäure und/oder deren Natrium-, Kalium oder Triethanolaminsalze (Gesamtmenge) 1:1,2 bis 1,2:1 beträgt,
die Gesamtmenge an Titandioxidpigmenten und 2-Phenylbenzimidazol-5-sulfonsäure und/oder deren Natrium-, Kalium oder Triethanolaminsalzen 2,5 bis 3,5 Gewichts-%, bezogen auf das Gesamtgewicht der Zubereitung beträgt und die Zubereitung das Verdickersystem in einer Konzentration von 0,35 bis 0,45 Gewichts-%, bezogen auf das Gesamtgewicht der Zubereitung, enthält, **dadurch gekennzeichnet, dass** die Titandioxidpigmente mit Trimethoxycaprylylsilan beschichtet sind.

2. Zubereitung nach Anspruch 1, **dadurch gekennzeichnet, dass** die Zubereitung einen Lichtschutzfaktor (SPF) von größer oder gleich 50 aufweist.

3. Zubereitung nach einem der vorhergehenden Ansprüche, **dadurch gekennzeichnet, dass** die Zubereitung eine Viskosität von 300 bis 2000 mPaS aufweist.

4. Zubereitung nach einem der vorhergehenden Ansprüche, **dadurch gekennzeichnet, dass** die Titandioxidpigmente eine durchschnittliche Teilchengröße von 35 nm x 29 nm aufweisen.

5. Zubereitung nach einem der vorhergehenden Anspruche, **dadurch gekennzeichnet, dass** die Zubereitung in Form einer Öl-in-Wasser-Emulsion vorliegt.

6. Zubereitung nach einem der vorhergehenden Ansprüche, **dadurch gekennzeichnet, dass** die Zubereitung frei ist von 3-(4'-Methyl)benzylidenbornan-2-on (INCI: 4-Methylbenzylidene Camphor).

7. Zubereitung nach einem der vorhergehenden Ansprüche, **dadurch gekennzeichnet, dass** die Zubereitung frei ist von Caprylylglycol.

8. Zubereitungen nach einem der vorhergehenden Ansprüche, **dadurch gekennzeichnet, dass** die Zubereitung eine UVA-Balance von größer oder gleich 20 aufweist.

9. Zubereitung nach einem der vorhergehenden Ansprüche, **dadurch gekennzeichnet, dass** die Zubereitung ein oder mehrere weitere UV-Filter gewählt aus der Gruppe der Verbindungen Phenylen-1,4-bis-(2-benzimidazyl)-3,3'-5,5'-tetrasulfonsäuresalze; 1,4-di(2-oxo-10-sulfo-3-bomylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmaion-sauredi(2-ethylhexyl)ester 4-Methoxyzimtsäure(2-ethylhexyl)ester; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-yl-1,3,5-triazin; Merocyanine; Zinkoxid enthält.

10. Kosmetische Zubereitung nach einem der vorhergehenden Ansprüche, **dadurch gekennzeichnet, dass** die Zubereitung Antioxidantien enthält.

## Claims

1. Cosmetic preparation comprising
titanium dioxide pigments,
a thickener system of polymeric thickeners, which are crosslinked in each case,
2-phenylbenzimidazole-5-sulphonic acid and/or sodium, potassium or triethanolamine salts thereof,
where
the ratio of titanium dioxide pigments to 2-phenylbenzimidazole-5-sulphonic acid and/or sodium, potassium or triethanolamine salts thereof (total amount) is 1:1.2 to 1-2:1,
the total amount of titanium dioxide pigments and 2-phenylbenzimidazole-5-sulphonic acid and/or sodium, potassium or triethanolamine salts thereof is 2.5 to 3.5% by weight, based on the total weight of the preparation, and the preparation comprises the thickener system in a concentration of from 0.35 to 0.45% by weight, based on the total weight of the preparation, **characterized in that** the titanium dioxide pigments are coated with trimethoxycaprylysilane.

2. Preparation according to Claim 1, **characterized in that** the preparation has a sun protection factor (SFP) of greater than or equal to 50.

3. Preparation according to one of the preceding claims, **characterized in that** the preparation has a viscosity of from 300 to 2000 mPaS.

4. Preparation according to one of the preceding claims, **characterized in that** the titanium dioxide pigments have an average particle size of 35 nm x 29 nm.

5. Preparation according to one of the preceding claims, **characterized in that** the preparation is present in the form of an oil-in-water emulsion.

6. Preparation according to one of the preceding claims, **characterized in that** the preparation is free from 3-(4'-methyl)benzylidenebornan-2-one (INCI: 4-Methylbenzylidene Camphor).

7. Preparation according to one of the preceding claims, **characterized in that** the preparation is free from caprylyl glycol.

8. Preparations according to one of the preceding claims, **characterized in that** the preparation has a UVA balance of greater or equal to 20.

9. Preparation according to one of the preceding claims, **characterized in that** the preparation comprises one or more further UV filters selected from the group of the compounds phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor;
3-benzylidenecamphor;
ethylhexyl salicylate; terephthalidenedicamphorsulphonic acid; 2-ethylhexyl 4-(dimethylamino)benzoate;
amyl 4-(dimethylamino)benzoate;
di(2-ethylhexyl) 4-methoxybenzalmalonate;
2-ethylhexyl 4-methoxycinnamate;
isopentyl 4-methoxycinnamate;
2-hydroxy-4-methoxybenzophenone, 2-hydroxy-4-methoxy-4'-methylbenzophenyne; 2,2'-dihydroxy-4-methoxybenzophenone,
hexyl 2-(4'-diethylamino-2'-hydroxybenzoyl)benzoate; 4-(tert-butyl)-4'-methoxydibenzoylmethane; homomenthyl salicylate; 2-ethylhexyl 2-hydroxybenzoate;
2-ethylhexyl 2-cyano-3,3-diphenylacrylate; dimethicodiethyl benzalmalonate; 3-(4-(2,2-bis-ethoxycarbonylvinyl)phenoxy)propenyl)methoxy-siloxane/dimethylsiloxane copolymer; 2,4-bis([4-(2-ethylhexyloxy)-2-hydroxy]phenyl)-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone);
2,4-bis[5-1(dimethylpropyl)benzoxazol-2-yl-(4-phenyl)imino]-6-(2-ethylhexyl)imino-1 ,3,5-triazine with the CAS NO. 288254-16-0; tris(2-ethylhexyl) 4,4',4"-(1,3,5-triazine-2,4,8-triyltriimino)trisbenzoate (also 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-traizine; merocyanines, zinc oxide.

10. Cosmetic preparation according to one of the preceding claims, **characterized in that** the preparation comprises antioxidants.

## Revendications

1. Préparation cosmétique contenant des pigments dioxyde de titane,
un système épaississant à base d'épaississants polymères, qui sont chacun réticulés,
de l'acide 2-phénylbenzimidazole-5-sulfonique et/ou ses sels de sodium, potassium ou triéthanolamine,
le rapport des pigments dioxyde de titane à l'acide 2-phénylbenzimidazole-5-sulfonique et/ou à ses sels de sodium, potassium ou triéthanolamine (quantité totale) valant de 1:1,2 à 1,2:1,
la quantité totale de pigments dioxyde de titane et d'acide 2-phénylbenzimidazole-5-sulfonique et/ou de ses sels de sodium, potassium ou triéthanolamine valant de 2.5 à 3,5 % en poids, par rapport au poids total de la préparation et la préparation contenant le système épaississant à une concentration de 0,35 à 0,45 % en poids, par rapport au poids total de la préparation, **caractérisée en ce que** les pigments dioxyde de titane sont enrobés de triméthoxycaprylsilane.

2. Préparation selon la revendication 1, **caractérisée en ce que** la préparation présente un facteur photoprotecteur (SPF) supérieur ou égal à 50.

3. Préparation selon l'une quelconque des revendications précédentes, **caractérisée en ce que** la préparation présente une viscosité de 300 à 2 000 mPa.s.

4. Préparation selon l'une quelconque des revendications précédentes, **caractérisée en ce que** les pigments dioxyde de titane présentent une taille moyenne de particule de 35 nm x 29 nm.

5. Préparation selon l'une quelconque des revendications précédentes, **caractérisée en ce que** la préparation se trouve sous forme d'une émulsion huile-dans-eau.

6. Préparation selon l'une quelconque des revendications précédentes, **caractérisée en ce que** la préparation est exempte de 3-(4'-méthyl)benzylidène-bornan-2-one (INCI : 4-methylbenzylidone camphor).

7. Préparation selon l'une quelconque des revendications précédentes, **caractérisée en ce que** la préparation est exempte de caprylyglycol.

8. Préparation selon l'une quelconque des revendications précédentes, **caractérisée en ce que** la préparation présente un rapport UVA supérieur ou égal à 20.

9. Préparation selon l'une quelconque des revendications précédentes, **caractérisée en ce que** la préparation contient un ou plusieurs autres filtres UV choisis dans le groupe des composés sels d'acide phénylène-1,4-bis-(2-benzimidazyl)-3,3'-5,5'-tétrasulfonique ; 1,4-di(2-oxo-10-sulfo-3-bornylidène-méthyl)-benzène et ses sels ; sels d'acide 4-(2-oxo-3-bornylidèneméthyl)-benzènesulfonique ; sels d'acide 2-méthyl-5-(2-oxo-3-bornylidèneméthyl)-sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphosulfonique ; 4-(diméthylamino)-benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isopentyle ; 2-hydroxy-4-méthoxybenzophénone, 2-hydroxy-4-méthoxy-4'-méthylbenzophénone ; 2,2'-dihydroxy-4-méthoxybenzophénone ; 2-(4'-diéthylamino-2'-hydroxybenzoyl)-benzoate d'hexyle, 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère 3-(4-(2,2-bis-éthoxycarbonylvinyl)-phénoxy)-propényl)-méthoxysiloxane / diméthylsiloxane ; 2,4-bis-{[4-(2-éthyl-hexyloxy)-2-hydroxy]-phényl}-6-(4-méthoxyphényl)-1,3,5-triazine (INCI : bis-ethylhexyloxyphenol methoxyphenyl triazin); dioctylbutylamidotriazone (INCI : diethylhexyl-butamidotriazone) ; 2,4-bis-[5-1(dimethylpropyl)benzoxazol-2-yl-(4-phényl)-imino]-6-(2-éthylhexyl)-imino-1,3,5-triazine portant le n° CAS 288254-16-0 ; 4,4',4"-(1,3,5-triazin-2,4,6-triyltriimino)-tris-benzoate de tris(2-éthylhexyle) (également : 2,4,6-tris-[anilino-(p-carbo-2'-éthyl-1'-hexyloxy)]-1,3,5-triazine (INCI : ethylhexyl triazone) ; 2,4,6-tribiphényl-4-yl-1,3,5-triazine ; mérocyanine ; oxyde de zinc.

10. Préparation cosmétique selon l'une quelconque des revendications précédentes, **caractérisée en ce que** la préparation contient des antioxydants.
